# EUROPEAN PATENT APPLICATION

(11) **EP 4 851 135 A2**
(43) Date of publication of application: **09.12.2026**
(21) Application number: 26213488.5
(22) Date of filing: 13.05.2021
(51) Int. Cl.: C07C 237/52

(54) **MYRISTOYL DERIVATIVES OF 9-AMINO-DOXYCYCLINE FOR TARGETING CANCER STEM CELLS AND PREVENTING METASTASIS**

(30) Priority: 13.05.2020 US 202063024216 P
(62) Document number(s) of the earlier application(s) in accordance with Art. 76 EPC: 21805219.9 / 4 153 563
(71) Applicant: Lunella Biotech, Inc., Ottawa, ON K1Z 1A1 (CA)
(72) Inventor: LISANTI, Michael, P., Manchester M20 2DS (GB); SOTGIA, Federica, Manchester M20 2DS (GB); OZSVARI, Béla, Manchester M38 9LD (GB); KANGASMETSA, Jussi, Cambridge CB2 9BX (GB)
(74) Representative: Mathys & Squire

(57) **Abstract**

Disclosed are 9-amino-doxycycline derivatives that target cancer stem cells and inhibit cancer metastasis. These compounds selectively target CSCs, potently inhibit tumor cell metastasis in vivo, with little or no toxicity, and minimize the risk of driving antibiotic resistance. In one embodiment, a 14 carbon fatty acid moiety is covalently attached to the free amino group of 9-amino-doxycycline. The resulting "Doxy-Myr" conjugate is over 5 -fold more potent than doxycycline for inhibiting the anchorage- independent growth of MCF7 CSCs. Doxy-Myr did not affect the viability of the total MCF7 cancer cell population or normal fibroblasts grown as 2D-monolayers, showing remarkable selectivity for CSCs. Doxy-Myr did not show antibiotic activity, against Escherichia coli and Staphylococcus aureus. Conjugates having either longer (16 carbon; palmitic acid) or shorter (12 carbon; lauric acid) fatty acid chain lengths had similar activity.

## Description

### RELATED APPLICATIONS

This application claims the benefit of U.S. provisional patent application 63/024,216, filed May 13, 2020, and incorporated herein by reference in its entirety.

### FIELD

The present disclosure relates to inhibiting mitochondrial function and eradicating cancer, and in particular inhibiting cancer stem cells (CSCs) and preventing or reducing the likelihood of metastasis, using derivatives of 9-amino-doxycycline.

### BACKGROUND

Researchers have struggled to develop new anti-cancer treatments. Conventional cancer therapies (e.g. irradiation, alkylating agents such as cyclophosphamide, and anti-metabolites such as 5-Fluorouracil) have attempted to selectively detect and eradicate fast-growing cancer cells by interfering with cellular mechanisms involved in cell growth and DNA replication. Other cancer therapies have used immunotherapies that selectively bind mutant tumor antigens on fast-growing cancer cells (e.g., monoclonal antibodies). Unfortunately, tumors often recur following these therapies at the same or different site(s), indicating that not all cancer cells have been eradicated. Relapse may be due to insufficient chemotherapeutic dosage and/or emergence of cancer clones resistant to therapy. Hence, novel cancer treatment strategies are needed.

Advances in mutational analysis have allowed in-depth study of the genetic mutations that occur during cancer development. Despite having knowledge of the genomic landscape, modern oncology has had difficulty with identifying primary driver mutations across cancer subtypes. The harsh reality appears to be that each patient's tumor is unique, and a single tumor may contain multiple divergent clone cells. What is needed, then, is a new approach that emphasizes commonalities between different cancer types. Targeting the metabolic differences between tumor and normal cells holds promise as a novel cancer treatment strategy. An analysis of transcriptional profiling data from human breast cancer samples revealed more than 95 elevated mRNA transcripts associated with mitochondrial biogenesis and/or mitochondrial translation. Additionally, more than 35 of the 95 upregulated mRNAs encode mitochondrial ribosomal proteins (MRPs). Proteomic analysis of human breast cancer stem cells likewise revealed the significant overexpression of several mitoribosomal proteins as well as other proteins associated with mitochondrial biogenesis.

Mitochondria are extremely dynamic organelles in constant division, elongation and connection to each other to form tubular networks or fragmented granules in order to satisfy the requirements of the cell and adapt to the cellular microenvironment. The balance of mitochondrial fusion and fission dictates the morphology, abundance, function and spatial distribution of mitochondria, therefore influencing a plethora of mitochondrial-dependent vital biological processes such as ATP production, mitophagy, apoptosis, and calcium homeostasis. In turn, mitochondrial dynamics can be regulated by mitochondrial metabolism, respiration and oxidative stress. Thus, it is not surprising that an imbalance of fission and fusion activities has a negative impact on several pathological conditions, including cancer. Cancer cells often exhibit fragmented mitochondria, and enhanced fission or reduced fusion is often associated with cancer, although a comprehensive mechanistic understanding on how mitochondrial dynamics affects tumorigenesis is still needed.

An intact and enhanced metabolic function is necessary to support the elevated bioenergetic and biosynthetic demands of cancer cells, particularly as they move toward tumor growth and metastatic dissemination. Not surprisingly, mitochondria-dependent metabolic pathways provide an essential biochemical platform for cancer cells, by extracting energy from several fuels sources.

Cancer stem-like cells are a relatively small sub-population of tumor cells that share characteristic features with normal adult stem cells and embryonic stem cells. As such, CSCs are thought to be a 'primary biological cause' for tumor regeneration and systemic organismal spread, resulting in the clinical features of tumor recurrence and distant metastasis, ultimately driving treatment failure and premature death in cancer patients undergoing chemo- and radio-therapy. Evidence indicates that CSCs also function in tumor initiation, as isolated CSCs experimentally behave as tumor-initiating cells (TICs) in pre-clinical animal models. As approximately 90% of all cancer patients die pre-maturely from metastatic disease world-wide, there is a great urgency and unmet clinical need, to develop novel therapies for effectively targeting and eradicating CSCs. Most conventional therapies do not target CSCs and often increase the frequency of CSCs, in the primary tumor and at distant sites.

Recently, energetic metabolism and mitochondrial function have been linked to certain dynamics involved in the maintenance and propagation of CSCs, which are a distinguished cell sub-population within the tumor mass involved in tumor initiation, metastatic spread and resistance to anti-cancer therapies. For instance, CSCs show a peculiar and unique increase in mitochondrial mass, as well as enhanced mitochondrial biogenesis and higher activation of mitochondrial protein translation. These behaviors suggest a strict reliance on mitochondrial function. Consistent with these observations, an elevated mitochondrial metabolic function and OXPHOS have been detected in CSCs across multiple tumor types.

One emerging strategy for eliminating CSCs exploits cellular metabolism. CSCs are among the most energetic cancer cells. Under this approach, a metabolic inhibitor is used to induce ATP depletion and starve CSCs to death. So far, the inventors have identified numerous FDA-approved drugs with off-target mitochondrial side effects that have anti-CSC properties and induce ATP depletion, including, for example, the antibiotic Doxycycline, which functions as a mitochondrial protein translation inhibitor. Doxycycline, a long-acting Tetracycline analogue, is currently used for treating diverse forms of infections, such as acne, acne rosacea, and malaria prevention, among others. In a recent Phase II clinical study, pre-operative oral Doxycycline (200 mg/day for 14 days) reduced the CSC burden in early breast cancer patients between 17.65% and 66.67%, with a near 90% positive response rate.

However, certain limitations restrain the use of sole anti-mitochondria agents in cancer therapy, as adaptive mechanisms can be adopted in the tumor mass to overcome the lack of mitochondrial function. These adaptive mechanisms include, for example, the ability of CSCs to shift from oxidative metabolism to alternate energetic pathways, in a multi-directional process of metabolic plasticity driven by both intrinsic and extrinsic factors within the tumor cells, as well as in the surrounding niche. Notably, in CSCs the manipulation of such metabolic flexibility can turn as advantageous in a therapeutic perspective. What is needed, then, are therapeutic approaches that either prevent these metabolic shifts, or otherwise take advantage of the shift to inhibit cancer cell proliferation.

Further, various anti-cancer agents have been described that also have some degree of antibiotic activity. For example, various repurposed antibiotics have been identified as having CSC inhibition properties. While such compounds have potential use as part of cancer therapy, they raise concerns about increases in antibiotic resistance. Thus, what is needed are therapeutic options that do not possess antibiotic activity, and are therefore unlikely to contribute to antibiotic resistance.

An object of this disclosure is to describe pharmaceutical compounds designed for specifically targeting and eradicating cancer cells and, more particularly, CSCs.

It is another object of this disclosure to describe pharmaceutical compounds designed for specifically targeting CSCs involved in metastasis and tumor recurrence, and having no antibiotic activity.

It is another object of this disclosure to identify new anti-cancer therapeutic approaches and treatments, and, more particularly, for preventing and/or reducing the likelihood of metastasis and tumor recurrence.

### SUMMARY

The present approach relates to a family of 9-amino-Doxycycline derivatives that specifically target cancer stem cells, and inhibit cancer metastasis and recurrence. The compounds disclosed herein potently inhibit tumor cell metastasis in vivo, with little or no toxicity. These compounds selectively target CSCs while effectively minimizing the risk of driving antibiotic resistance, and are suitable for therapeutic use for preventing and/or reducing the likelihood of metastasis and recurrence. In one embodiment, a 14 carbon fatty acid moiety is covalently attached to the free amino group of 9-amino-Doxycycline. The resulting "Doxy-Myr" conjugate is over 5-fold more potent than doxycycline, in terms of IC50 for inhibiting the anchorage-independent growth of MCF7 breast CSCs. Doxy-Myr did not affect the viability of the total MCF7 cancer cell population or normal fibroblasts grown as 2D-monolayers, showing remarkable selectivity for CSCs. Using both gram-negative and gram-positive bacterial strains, Doxy-Myr did not show antibiotic activity, against Escherichia coli and Staphylococcus aureus. Thus, compounds of the present approach are not likely to cause antibacterial resistance to front-line antibiotic Doxycycline. Conjugates having either longer (16 carbon; palmitic acid) or shorter (12 carbon; lauric acid) fatty acid chain lengths had similar activity, but were less potent than Doxy-Myr for the targeting of CSCs.

The present approach relates to the chemical synthesis and biological activity of new 9-amino-Doxycycline derivatives, modified with a fatty acid moiety at the 9-position to increase the effectiveness in the targeting of CSCs and preventing and reducing the likelihood of metastasis. Embodiments of the present approach are compounds having the general formula shown below, in which R is a C₄-C₁₈ alkyl, and preferably a linear alkyl, and preferably a saturated alkyl, or a pharmaceutically acceptable salt thereof (e.g., monohydrate, hyclate, etc.).

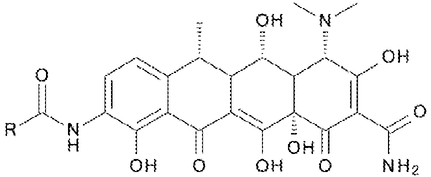

The present approach may take the form of a compound having the general formula: 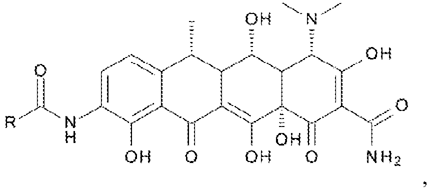 wherein R is a linear, saturated alkyl having from 4 to 18 carbons, or a pharmaceutically acceptable salt thereof. In some embodiments, R is a linear, saturated alkyl having from 11 to 16 carbons. For example, in some embodiments the compound may have the formula:

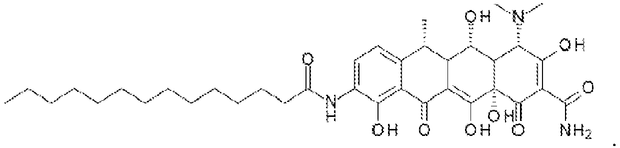

In some embodiments, the compound may have the formula:

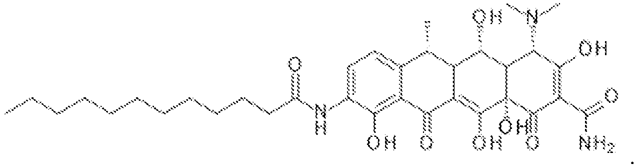

In some embodiments, the compound may have the formula:

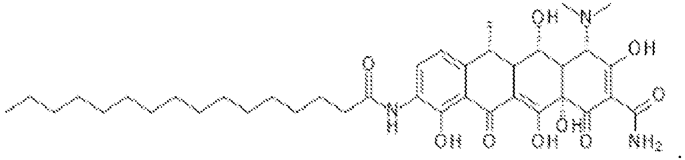

In embodiments in which the compound is a pharmaceutically acceptable salt, the salt may be, for example, one of monohydrate and hyclate.

The present approach may also take the form of a pharmaceutical composition having a compound with the general formula: 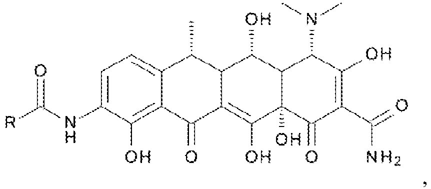 wherein R is a linear, saturated alkyl having from 4 to 18 carbons, or a pharmaceutically acceptable salt thereof, and a pharmaceutically acceptable carrier. In some embodiments, R may be a linear, saturated alkyl having from 11 to 16 carbons. For example, R may be 11, 13, or 15. The pharmaceutically acceptable carrier may include one or more of a sugar, a starch, cellulose, an excipient, an oil, a glycol, a polyol, an ester, an agar, and a buffering agent. It should be appreciated that the person having an ordinary level of skill in the art can determine an appropriate pharmaceutically acceptable carrier without undue burden, using ordinary means available in the art.

In some embodiments, the pharmaceutical composition may be for use in one of preventing metastasis, reducing inflammation, reducing fibrosis, and reducing viral replication.

The present approach may also take the form of methods for preventing metastasis in a patient, the method comprising administering to the patient a pharmaceutically effective amount of a compound as described herein.

The present approach may also take the form of methods for reducing inflammation in a patient, the method comprising administering to the patient a pharmaceutically effective amount of a compound as described herein.

The present approach may also take the form of methods for reducing fibrosis in a patient, the method comprising administering to the patient a pharmaceutically effective amount of a compound as described herein.

The present approach may also take the form of methods for reducing virus replication in a patient, the method comprising administering to the patient a pharmaceutically effective amount of a compound as described herein.

These and other embodiments will be apparent to the person having an ordinary level of skill in the art in view of this description, the claims appended hereto, and the applications incorporated by reference herein.

### BRIEF DESCRIPTION OF THE DRAWINGS

Figure 1 shows the chemical structures of demonstrative 9-amino-Doxycycline derivatives, (A) Doxy-Myr and (B) Doxy-TPP.
Figure 2 shows 3-D mammosphere formation assay results for an embodiment of the present approach.
Figure 3 shows comparative images of compounds fluorescing within cells.
Figures 4A and 4B show the cell viability results of treating MCF7 cells and normal human fibroblast cells (hTERT-BJ1) with Doxycycline ("Doxy") or Doxy-Myr.
Figures 5A-5D show the results of treatment with Doxycycline or Doxy-Myr, on MCF7 2d-monolyaer proliferation.
Figures 6A-6C show the results of the impact of treatment with Doxycycline or Doxy-Myr on cell cycle progression, in the form of representative FACS cell cycle profiles.
Figure 7 shows the results of Doxycycline (solid line), Doxy-Pal (short dashes), Doxy-Laur (alternative dash-ticks), and Doxy Myr (long dashes).
Figures 8A-8D show the antibiotic effects of Doxycycline, Doxy-Myr, Doxy-Laur, and Doxy-Pal (respectively), at various concentrations, against *E. coli* and *S. aureus.*
Figure 9 illustrates the CAM assay metastasis results.

### DESCRIPTION

The following description illustrates embodiments of the present approach in sufficient detail to enable practice of the present approach. Although the present approach is described with reference to these specific embodiments, it should be appreciated that the present approach can be embodied in different forms, and this description should not be construed as limiting any appended claims to the specific embodiments set forth herein. Rather, these embodiments are provided so that this disclosure will be thorough and complete, and will fully convey the scope of the present approach to those skilled in the art.

This description uses various terms that should be understood by those of an ordinary level of skill in the art. The following clarifications are made for the avoidance of doubt. The terms "treat," "treated," "treating," and "treatment" include the diminishment or alleviation of at least one symptom associated or caused by the state, disorder or disease being treated, in particular, cancer. In certain embodiments, the treatment comprises diminishing and/or alleviating at least one symptom associated with or caused by the cancer being treated, by the compound of the invention. In some embodiments, the treatment comprises causing the death of a category of cells, such as CSCs likely to be involved in metastasis or recurrence, of a particular cancer in a host, and may be accomplished through preventing cancer cells from further propagation, and/or inhibiting CSC function through, for example, depriving such cells of mechanisms for generating energy. For example, treatment can be diminishment of one or several symptoms of a cancer, or complete eradication of a cancer. As another example, the present approach may be used to inhibit mitochondrial metabolism in the cancer, eradicate (e.g., killing at a rate higher than a rate of propagation) CSCs in the cancer, eradicate TICs in the cancer, eradicate circulating tumor cells in the cancer, inhibit propagation of the cancer, target and inhibit CSCs, target and inhibit TICs, target and inhibit circulating tumor cells, prevent (i.e., reduce the likelihood of) metastasis, prevent recurrence, sensitize the cancer to a chemotherapeutic, sensitize the cancer to radiotherapy, sensitize the cancer to phototherapy.

The terms "cancer stem cell" and "CSC" refer to the subpopulation of cancer cells within tumors that have capabilities of self-renewal, differentiation, and tumorigenicity when transplanted into an animal host. Compared to "bulk" cancer cells, CSCs have increased mitochondrial mass, enhanced mitochondrial biogenesis, and higher activation of mitochondrial protein translation. As used herein, a "circulating tumor cell" is a cancer cell that has shed into the vasculature or lymphatics from a primary tumor and is carried around the body in the blood circulation. The CellSearch Circulating Tumor Cell Test may be used to detect circulating tumor cells.

The phrase "pharmaceutically effective amount," as used herein, indicates an amount necessary to administer to a host, or to a cell, tissue, or organ of a host, to achieve a therapeutic result, such as regulating, modulating, or inhibiting protein kinase activity, e.g., inhibition of the activity of a protein kinase, or treatment of cancer. A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

As used herein, the phrase "active compound" refers to the 9-amino-Doxycycline derivative compounds described herein, which may include a pharmaceutically acceptable salt or isotopic analog thereof. It should be appreciated that the active compound(s) may be administered to the subject through any suitable approach, as would be known to those having an ordinary level of skill in the art. It should also be appreciated that the amount of active compound and the timing of its administration may be dependent on the individual subject being treated (e.g., the age and body mass, among other factors), on the manner of administration, on the pharmacokinetic properties of the particular active compound(s), and on the judgment of the prescribing physician. Thus, because of subject to subject variability, any dosages described herein are intended to be initial guidelines, and the physician can titrate doses of the compound to achieve the treatment that the physician considers appropriate for the subject. In considering the degree of treatment desired, the physician can balance a variety of factors such as age and weight of the subject, presence of preexisting disease, as well as presence of other diseases. Pharmaceutical formulations can be prepared for any desired route of administration including, but not limited to, oral, intravenous, or aerosol administration, as discussed in greater detail below.

The phrase "pharmaceutically acceptable carrier" as used herein, means a pharmaceutically acceptable material, composition, or vehicle, such as a liquid or solid filler, diluent, excipient, solvent, or encapsulating material. Each carrier must be "acceptable" in the sense of being compatible with the other ingredients of the formulation and not injurious to the patient. Some examples of materials which can serve as pharmaceutically acceptable carriers include: (1) sugars, such as lactose, glucose and sucrose: (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

As used herein, the term derivative is a chemical moiety derived or synthesized from a referenced chemical moiety. For example, compounds according to the present approach may be referred to as 9-amino-Doxycycline derivatives, and have a fatty acid moiety conjugated at the 9-position. As used herein, a conjugate is a compound formed by the joining of two or more chemical compounds. For example, a conjugate of doxycycline and a fatty acid results in a compound having a doxycycline moiety and a moiety derived from the fatty acid As used herein, a fatty acid is a carboxylic acid with an aliphatic chain, which is either saturated or unsaturated. Examples of fatty acids include short chain fatty acids (i.e., having 5 or fewer carbon atoms in the chemical structure), medium-chain fatty acids (having 6-12 carbon atoms in the chemical structure), and other long chain fatty acids (i.e., having 13-21 carbon atoms in the chemical structure). Examples of saturated fatty acids include lauric acid (CH₃(CH₂)₁₀COOH), palmitic acid (CH₃(CH₂)₁₄COOH), stearic acid (CH₃(CH₂)₁₆COOH), and myristic acid (CH₃(CH₂)₁₂COOH). Oleic acid (CH3(CH2)7CH=CH(CH2)7COOH) is an example of a naturally occurring unsaturated fatty acid. It should be appreciated that compounds of the present approach involve 9-amino-Doxycycline conjugated with a linear, saturated fatty acid at the 9-position, and preferably a linear, saturated fatty acid having from 5 to 19 carbon atoms, and more preferably from 10 to 18 carbon atoms, and even more preferably, from 12 to 16 carbon atoms. In preferred embodiments, the linear, saturated fatty acid is myristic acid, having 14 carbon atoms.

The present approach relates to the chemical synthesis and biological activity of new 9-amino-Doxycycline derivatives, modified with a fatty acid moiety at the 9-position to increase the effectiveness in the targeting of CSCs and preventing and reducing the likelihood of metastasis. Embodiments of the present approach are compounds having the general formula [1], in which R is a C₄-C₁₈ alkyl, and preferably a linear alkyl, and preferably a saturated alkyl, or a pharmaceutically acceptable salt thereof (e.g., monohydrate, hyclate, etc.).

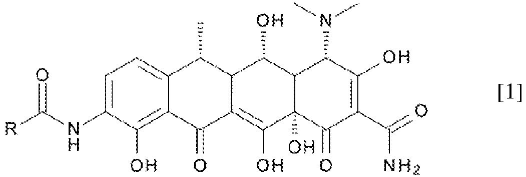

In a preferred embodiment, the compound is a 9-amino-Doxycycline derivative in which a myristic acid (14 carbon) moiety is covalently attached to the free amino group of 9-amino-Doxycycline, at the 9-position. The resulting compound is referenced herein as "Doxy-Myr" for brevity, shown below as compound [1A]. Other demonstrative preferred embodiments include a 9-amino-Doxycycline derivative in which a lauric acid (12 carbon) moiety is attached to the amino group at the 9-position, and a 9-amino-Doxycycline derivative in which a palmitic acid (16 carbon) moiety is attached to the amino group at the 9-position.

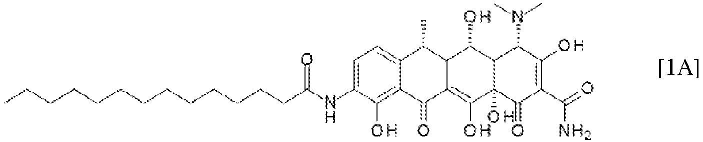

Various data is disclosed herein demonstrating the potency of Doxy-Myr, using the 3D-mammosphere assay, and its inhibitory effects on the anchorage-independent propagation of breast CSCs. Overall, Doxy-Myr is more than 5-fold more potent than Doxycycline. Moreover, Doxy-Myr showed better intracellular retention, and was specifically localized within a peri-nuclear membranous compartment. In contrast, when MCF7 breast cancer cells or normal fibroblasts were grown as 2D-monolayers, Doxy-Myr did not reveal any effects on cell viability or proliferation. This highlights the compound's unique selectivity for targeting the 3D-propagation of CSCs. Using MDA-MB-231 cells in the CAM assay, Doxy-Myr was found to potently inhibit tumor cell metastasis *in vivo,* with little or no chick embryo toxicity. Similar effects resulted from other 9-amino-Doxycycline conjugates, having longer alkyl chains (e.g., 16 carbon, palmitic acid) and shorter alkyl chains (e.g., 12 carbon, lauric acid). While effective, the data demonstrated that the conjugate having a 14-carbon alkyl chain, Doxy-Myr, was the most potent with respect to targeting of CSCs.

The data discussed herein show that lipophilic amide substituents on the 9-position of the tetracycline skeleton led to the loss of its antibacterial activity. Previously published structure-activity relationship studies have shown that chemical modification of the tetracycline skeleton at the 9-position can be tolerated, leading to diverse antibacterial activity, as is exemplified by the antibiotic Tigecycline. The lipophilicity of the tetracyclines seems to play a key role in the biological potency of this family of drugs.

The improvement in the biological properties of the 9-amino-Doxycycline derivatives for targeting CSCs, and the associated loss of antimicrobial activity, make these new compounds extremely useful in cancer therapy, without raising concerns about antibiotic resistance or deleterious effects on the human microbiome.

A previous study successfully used the parent compound, Doxycycline, to prevent bone metastasis in a mouse model, by employing MDA-MD-231 cells. Duivenvoorden WC, Popović SV, Lhoták S, Seidlitz E, Hirte HW, Tozer RG, Singh G. Doxycycline decreases tumor burden in a bone metastasis model of human breast cancer. Cancer Res. 2002 Mar 15;62(6):1588-91. However, the study did not examine the effects of Doxycycline on tumor growth, but only focused on bone metastasis. The study attributed the efficacy of Doxycycline to its tropism for bone and to its ability to act as a protease inhibitor for lysosomal cysteine proteinases, the cathepsins, and MMPs, because Doxycycline behaves as a zinc chelator.

In contrast, the present approach demonstrates that Doxycycline and 9-amino-Doxycycline derivatives such as Doxy-Myr act as inhibitors of metastasis, by targeting the 3D anchorage-independent growth of CSCs. This mechanism is a completely different molecular mechanism than bone metastasis. As such, based on these functional observations, it may be more appropriate to refer to tumor-spheres as metasta-spheres, to better reflect the close relationship between 3D anchorage-independent growth and metastasis.

Doxycycline is known to function as an inhibitor of the propagation of CSCs, through its ability to inhibit the small mitochondrial ribosome, which is an off-target side-effect. Normally, Doxycycline is used as a broad-spectrum antibiotic, with bacteriostatic properties, to fight a large number of infectious agents, including gram-negative and gram-positive bacteria. Therefore, the inventors sought to optimize the ability of Doxycycline for the targeting of CSCs, while minimizing its antibiotic activity, to derive a new chemical entity to selectively target CSCs.

Embodiments of the present approach use 9-amino-Doxycycline (shown below) as a scaffold. The 9-amino-Doxycycline compound, formally known as (4S,5S,6R,12aS)-9-amino-4-(dimethylamino)-3,5,10,12,12a-pentahydroxy-6-methyl-1,11-dioxo-4a,5,5a,6-tetrahydro-4H-tetracene-2-carboxamide, is a synthetic chemical often used in the synthesis of pharmaceutical compounds and other organic compounds. The amine group at the 9-position (of what is known in the art as the D-ring) is a useful substitution for the compounds of the present approach, and enables conjugates having little-to-no antibiotic activity.

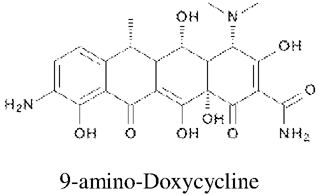

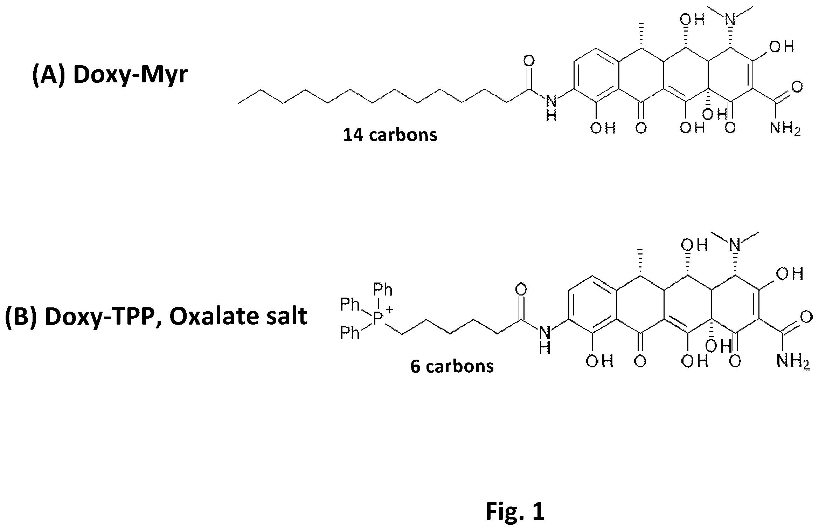

Substitutions according to the present approach may be made at the primary amine on the D-ring. In a first embodiment, the inventors covalently attached a linear, saturated 14-carbon fatty acid moiety (myristic acid) to 9-amino-Doxycycline at this location. This compound is referred to as Doxy-Myr. For comparative purposes, the inventors also synthesized a 6-carbon spacer arm terminating with tri-phenyl-phosphonium (TPP) to 9-amino-Doxycycline at the same location, referred to as Doxy-TPP. Figure 1 shows the chemical structures of these 9-amino-Doxycycline derivatives.

The addition of the fatty acid moiety (e.g., myristic acid) acts as a membrane targeting signal, leading to the increased retention of Doxy-Myr within membranous compartments, such as the plasma membrane, the endoplasmic reticulum (ER), the Golgi apparatus, and/or mitochondria. The TPP-moiety, in contrast, was expected to increase the membrane potential of the compound and target the compound to mitochondria in CSCs.

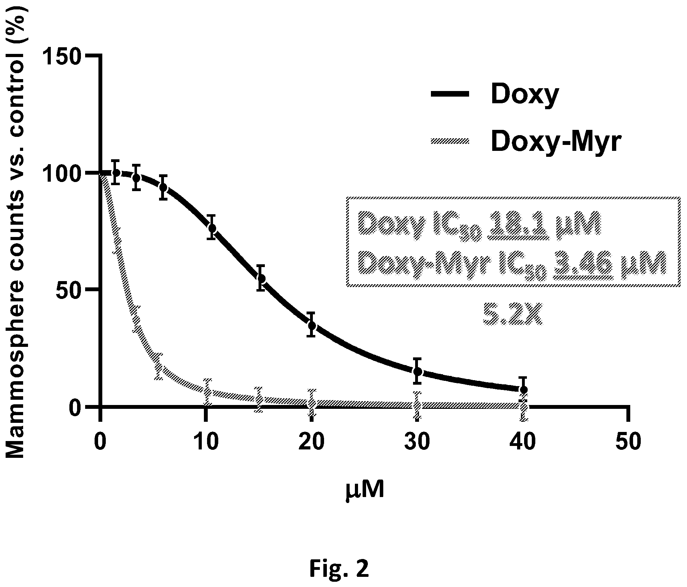

To determine the functional activity of the Doxy-Myr and Doxy-TPP compounds, the inventors used the 3D-mammosphere formation assay to assess each compound's ability to inhibit the anchorage-independent propagation of MCF7 CSCs. Figure 2 shows the results for both Doxycycline and Doxy-Myr. Doxy-Myr was >5-fold more potent than Doxycycline, with an IC₅₀ of 3.46 µM. In contrast, Doxycycline had an IC₅₀ of 18.1 µM. This demonstrates that Doxy-Myr is more potent for targeting the 3D anchorage-independent propagation of CSCs. Doxy-TPP was not more potent that Doxycycline itself, so further assays with Doxy-TPP were not carried out. The data for Doxy-TPP is not shown.

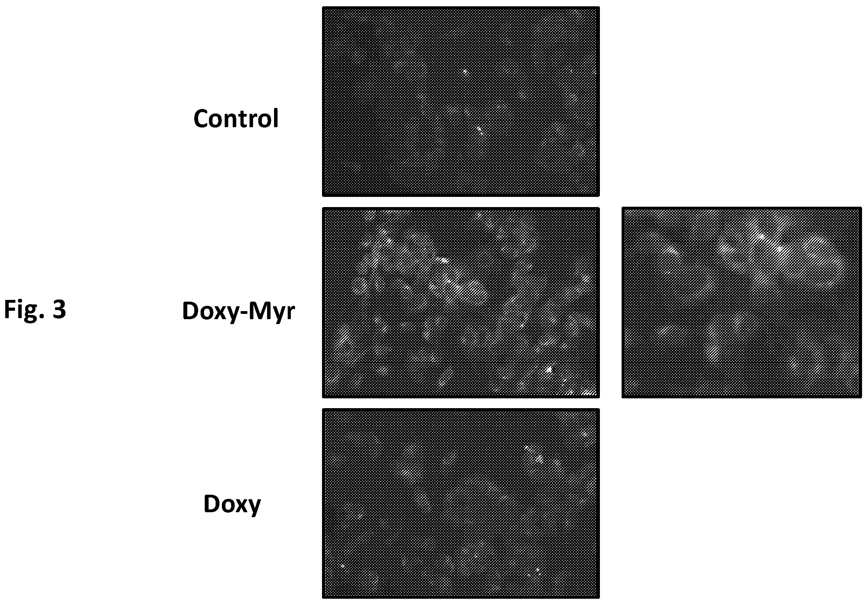

Further evaluation has demonstrated that Doxy-Myr has better retention within cells, compared to Doxycycline. Doxycycline and Doxy-Myr are fluorescent (Ex. 390 - 425 nm / Em. 520 - 560 nm), allowing for a visual comparison of cellular retention. Figure 3 shows images of the compounds fluorescing in monolayer MCF7 cells. As can be seen, Doxy-Myr is more easily detected and retained in monolayer MCF7 cells relative to both Doxycycline and cells treated with vehicle alone. Doxy-Myr fluorescence showed a peri-nuclear staining pattern, consistent with its partitioning and retention within intracellular membranous compartments. This observation could mechanistically explain its increased potency. No nuclear staining for Doxy-Myr was observed, indicating that the compound was predominantly excluded from the nucleus.

Embodiments of the present approach have been demonstrated to be non-toxic to normal fibroblasts. For example, the Doxy-Myr embodiment has been found to be non-toxic in 2D-monolayers of MCF7 cells or normal human fibroblasts. MCF7 cells and normal human fibroblasts (hTERT-BJ1) were treated over a period of 3 days to assess toxicity.

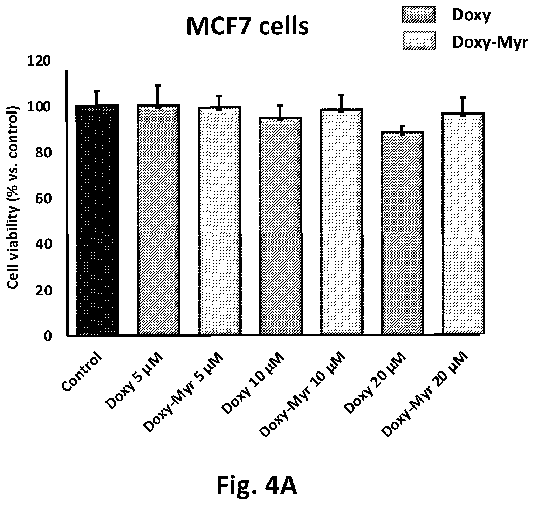

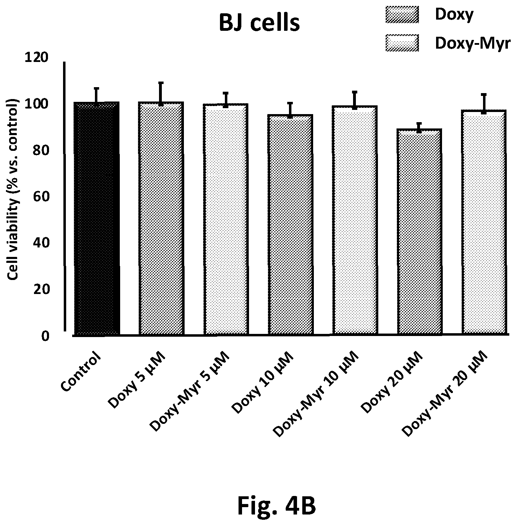

Figures 4A and 4B show the cell viability results of treating MCF7 cells and normal human fibroblast cells (hTERT-BJ1) with Doxycycline ("Doxy") or Doxy-Myr. The next cells were grown as 2D-monolayers, and were treated for a 3-day a period. At the concentrations tested, Doxy-Myr does not affect the viability of MCF7 cells or normal fibroblasts when grown as 2D-monolayers. As can be seen, both Doxycycline and Doxy-Myr had no appreciable effects on cell viability in either cell line, over the concentration range of 5 to 20 µM.

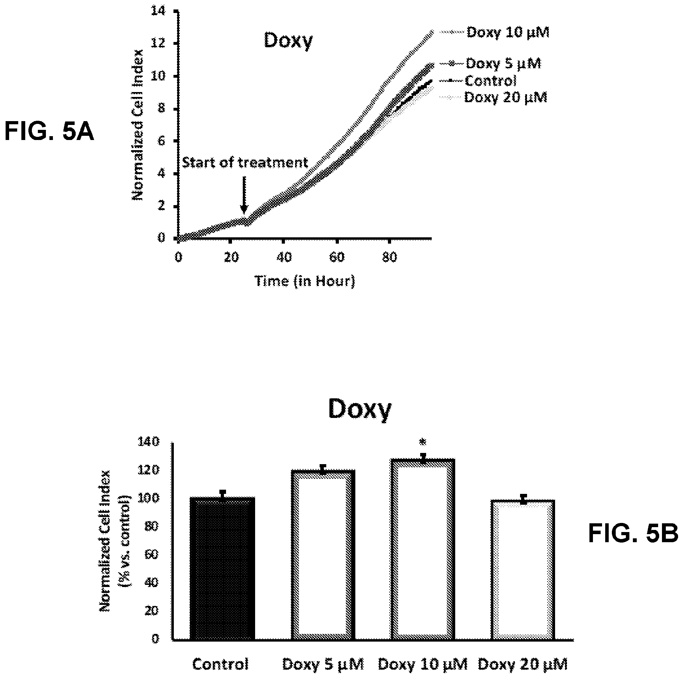

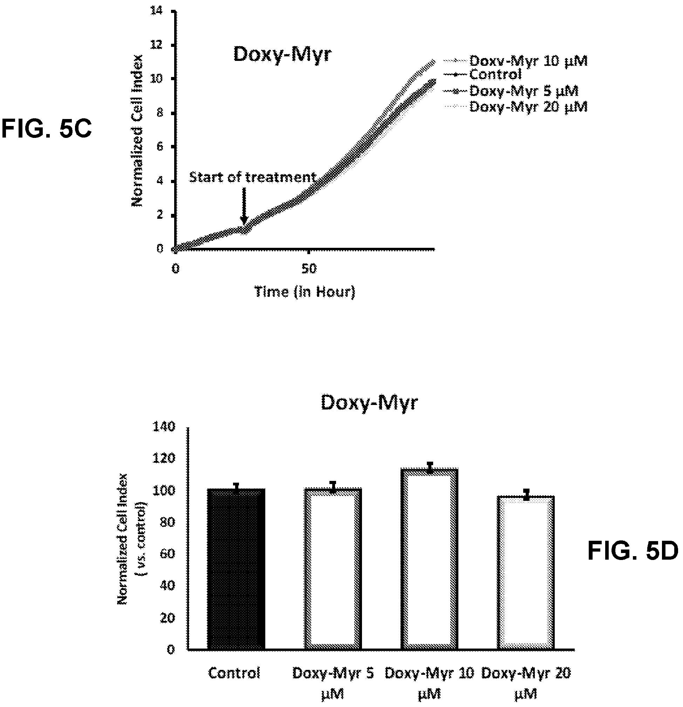

Potential 2D-effects on cell proliferation and the cell cycle were also determined using MCF7 cell monolayers. Figures 5A-5D show the results of treatment with Doxycycline or Doxy-Myr, on MCF7 2d-monolyaers, assessed using the xCELLigence. The results are shown relative to a control (no treatment). Figures 5A and 5B show results for Doxycycline, while Figures 5C and 5D show results for Doxy-Myr. As can be seen, treatment with either Doxycycline or Doxy-Myr did not inhibit the proliferation of MCF7 cells, relative to the control (no treatment).

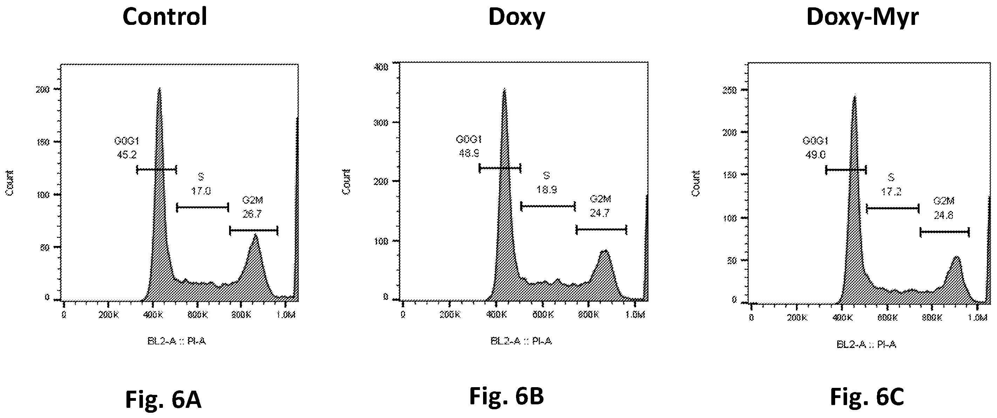

Figures 6A-6C show the results of the impact of treatment with Doxycycline or Doxy-Myr on cell cycle progression, in the form of representative FACS cell cycle profiles. MCF7 cells were cultured for 72 hours as 2D-monolayers, in the presence of Doxycycline (Fig. 6B) or Doxy-Myr (Fig. 6C), at a concentration of 10 µM. Vehicle-alone controls were processed in parallel (Fig. 6A). Relative to the parent compound Doxycycline, Doxy-Myr did not have any significant effects on reducing cell cycle progression in 2D-monolayers of MCF7 cells.

These results illustrate that, overall, Doxy-Myr did not significantly reduce cell viability, proliferation, or cell cycle progression of 2D-monolayers of MCF7 cells. This indicates that the effects of Doxy-Myr are specific for cell propagation under 3D anchorage-independent growth conditions.

Embodiments of the present approach have improved CSC inhibition effects, relative to Doxycycline. The data indicate that the effects are dependent on the length of the straight, saturated alkyl chain. Embodiments of 9-amino-Doxycycline conjugated with lauric acid (12-carbon chain, "Doxy-Laur") and palmitic acid (16-carbon chain, "Doxy-Pal") at the 9-position, shown below as compounds [1B] and [1C], respectively were synthesized and evaluated. Both 9-amino-Doxycycline conjugates were found to be less potent than Doxy-Myr in targeting CSCs.

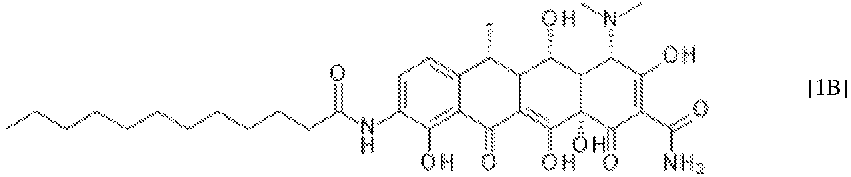

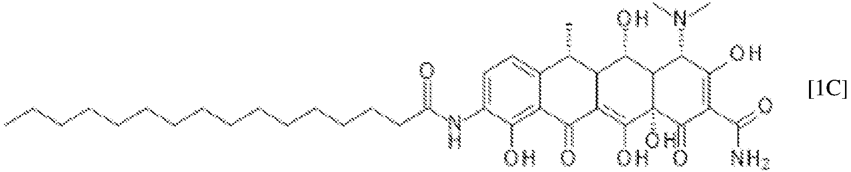

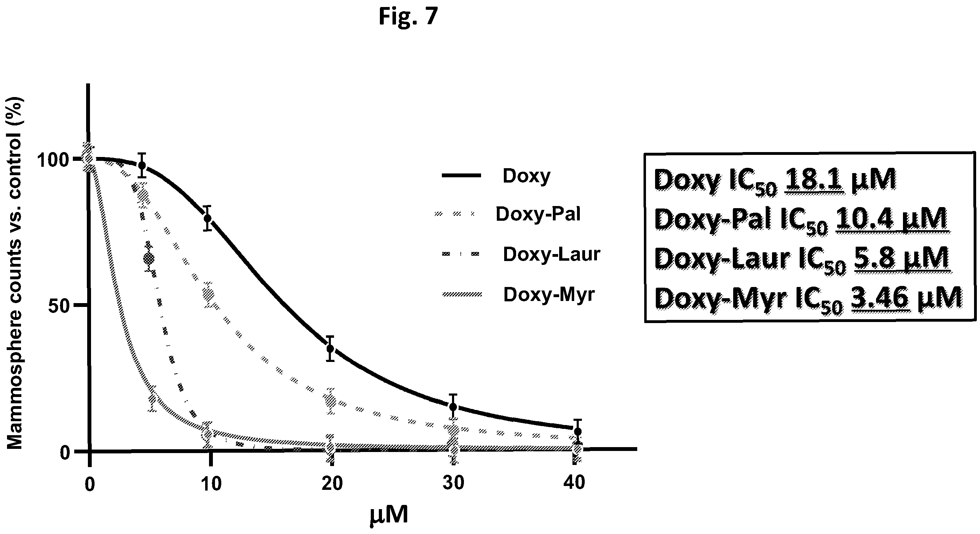

The 3D-mammosphere assay was used to compare the functional inhibitory activity of Doxycycline, Doxy-Myr, Doxy-Laur and Doxy-Pal, using MCF7 cells. Figure 7 shows the results of Doxycycline (solid line), Doxy-Pal (short dashes), Doxy-Laur (alternative dash-ticks), and Doxy Myr (long dashes). The results show that all three of the 9-amino-Doxycycline conjugates had improved inhibitory activity relative to Doxycycline. As can be seen, Doxy-Myr had an IC₅₀ of 3.46 µM, Doxy-Laur had an IC₅₀ of 5.8 µM, Doxy-Pal had an IC₅₀ of 10.4 µM, and Doxycycline had an IC₅₀ of 3.46 µM. Figure 7 demonstrates that embodiments of the present approach are effective at inhibiting the propagation of CSCs, and that the myristoyl derivatives of 9-amino-Doxycycline are the most potent. The rank order of potency is: Doxy-Myr > Doxy-Laur > Doxy-Pal > Doxycycline, with no direct correlation observed between chain length and activity. As such, conjugation with the 14-carbon myristic acid moiety appears to be the optimal chain length modification for embodiments of the present approach.

Embodiments of the present approach appear to lack antibiotic activity against common Gram-negative and Gram-positive bacteria. The lack of antibiotic activity would reduce or eliminate concerns about the potential development of antibiotic resistance, which may be a concern for using front-line antibiotics such as Doxycycline in connection with anti-cancer therapeutics. Doxycycline is a well-established, broad-spectrum antibiotic that is routinely used for therapeutically targeting both gram-negative and gram-positive bacterial infections. The antibiotic activity of fatty acid derivatives of 9-amino-Doxycycline of the present approach were evaluated.

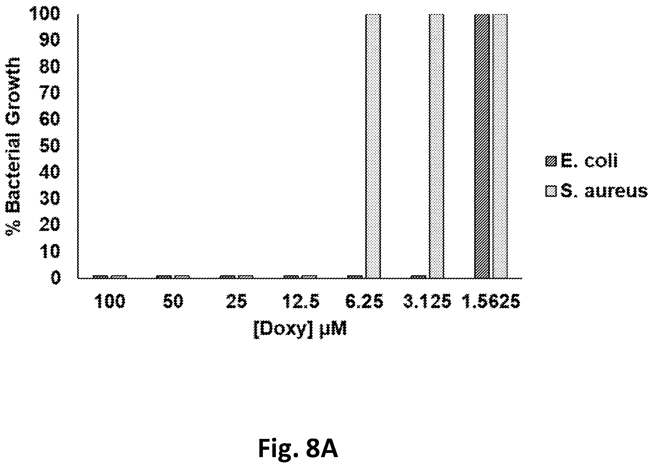

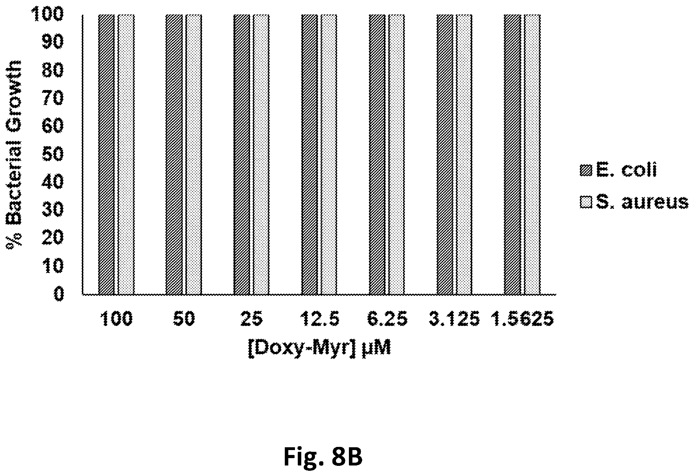

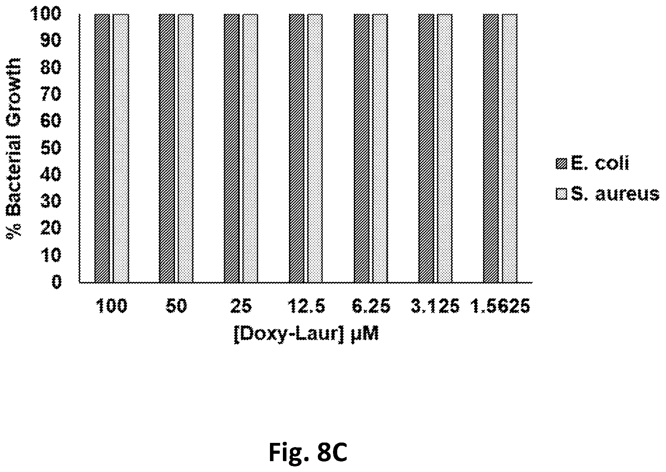

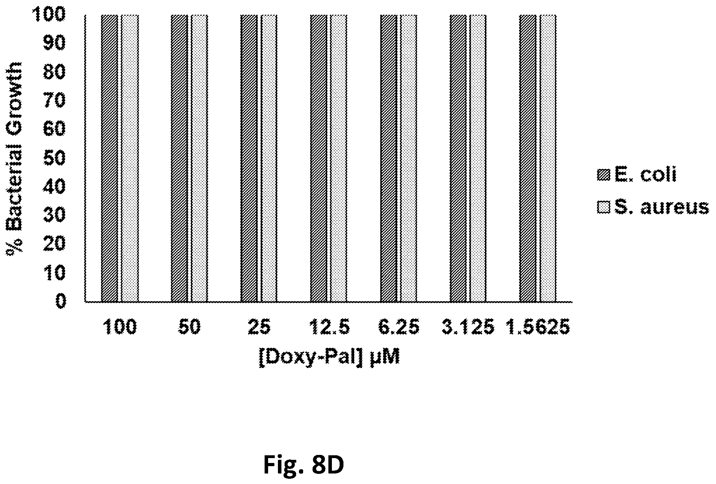

Figures 8A-8D show the antibiotic effects of Doxycycline, Doxy-Myr, Doxy-Laur, and Doxy-Pal (respectively), at various concentrations, against *E. coli* and *S. aureus.* As expected, Doxycycline potently and effectively inhibits the growth of both Gram-negative (*E. coli*) and Gram-positive (*S. aureus*) micro-organisms at most concentrations evaluated. However, in striking contrast, Doxy-Myr, Doxy-Laur and Doxy-Pal did not show any antibiotic activity across the same concentration ranges. Therefore, the chemical modifications to 9-amino-Doxycycline according to the present approach have removed any antibiotic activity, while simultaneously increasing the specificity for targeting and inhibiting CSCs.

Embodiments of the present approach inhibit cancer cell metastasis, without significant toxicity. These functional effects have been experimentally evaluated *in vivo.* MDA-MB-231 cells and the well-established chorio-allantoic membrane (CAM) assay in chicken eggs were used to quantitatively measure tumor growth and metastasis. MDA-MB-231 breast cancer cells were used for *in vivo* studies because they are estrogen-independent, intrinsically more aggressive, form larger tumors, and are significantly more migratory, invasive, and metastatic. As such, they are a better *in vivo* model, for simultaneously evaluating both tumor growth and spontaneous metastasis. Doxycycline has been shown to effectively inhibit the 3D anchorage-independent growth of MDA-MB-231 cells, making them ideal for evaluating embodiments of the present approach.

An inoculum of 1 X 10⁶ MDA-MB-231 cells was added onto the CAM of each egg (day E9) and then eggs were randomized into groups. On day E10, tumors were detectable and they were then treated daily for 8 days with vehicle alone (1% DMSO in PBS), Doxycycline, or Doxy-Myr. After 8 days of drug administration, on day E18 all tumors were weighed, and the lower CAM was collected to evaluate the number of metastatic cells, as analyzed by qPCR with specific primers for Human Alu sequences.

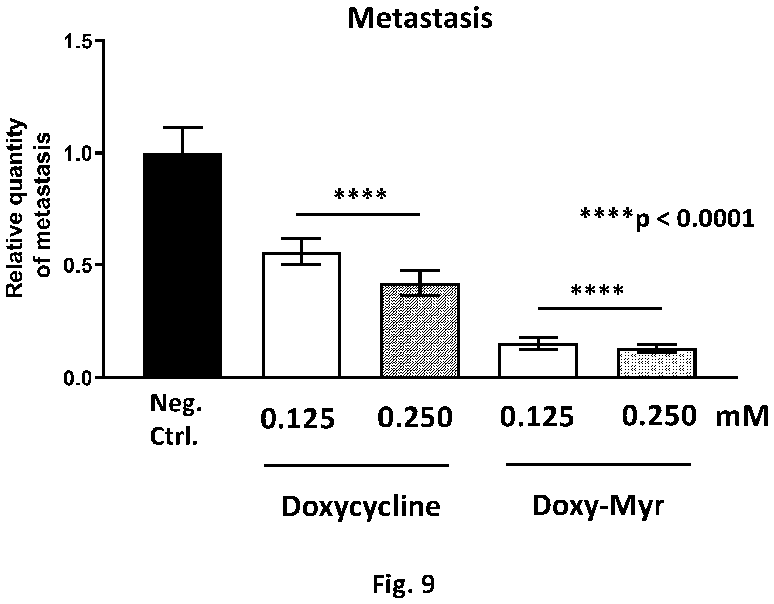

Both Doxycycline and Doxy-Myr showed significant effects on MDA-MB-231 cancer cell metastasis. Figure 9 illustrates the CAM assay metastasis results. The results are shown relative to the control (no treatment). As can be seen, Doxycycline inhibited metastasis by 44% to 57.5%. In contrast, Doxy-Myr inhibited metastasis by 85% to 87%, at the same concentrations tested for Doxycycline. This demonstrates that Doxy-Myr is significantly more effective than Doxycycline in terms of preventing or reducing the likelihood of metastasis.

Additionally, little-to-no embryo toxicity was observed for Doxycycline and Doxy-Myr in the CAM assay. Doxy-Myr has efficacy as an anti-metastatic agent, selectively inhibiting tumor metastasis, without significant toxicity or antibiotic activity. Table 1, below, summarizes the toxicity analysis from the CAM assay.

**Table 1. Chick Embryo Toxicity of Doxycycline and Doxy-Myr.**

| Group # | Group Description | Total | Alive | Dead | % Alive | % Dead |
|---|---|---|---|---|---|---|
| 1 | Neg. Ctrl. | 18 | 15 | 3 | 83.33 | 16.67 |
| 2 | Doxy, 0.125 mM | 13 | 11 | 2 | 84.62 | 15.38 |
| 3 | Doxy, 0.250 mM | 13 | 10 | 3 | 76.92 | 23.08 |
| 4 | Doxy-Myr, 0.125 mM | 14 | 11 | 3 | 78.57 | 21.43 |
| 5 | Doxy-Myr, 0.250 mM | 13 | 12 | 1 | 92.31 | 7.69 |

Although the data disclosed herein is predominantly based on breast cancer (e.g., MCF7 and hTERT-BJ1 cell lines), the compounds of the present approach have efficacy for other types of cancer. In prior work, the inventors demonstrated that mitochondrial biogenesis inhibitors successfully inhibited tumor-sphere formation in a wide-variety of cell lines from several tumor types. Table 4, below, lists cancer cell lines that have been shown to be susceptible to mitochondrial biogenesis inhibitors. Given these results, the present approach is effective for numerous cancer types.

**Table 2. Mitochondrial biogenesis inhibitors are effective against a wide variety of cancer types.**

| **Cancer Type** | **Cell Line(s)** |
|---|---|
| Breast (ER+) | MCF7 |
| | T47D |
| Breast (ER-) | MDA-MB-231 |
| DCIS | MCF10.DCIS.com ("pre-malignant") |
| | SKOV3 |
| Ovarian | Tov21G |
| | ES2 |
| Prostate | PC3 |
| Pancreatic | MIA PaCa2 |
| Lung | A549 |
| Melanoma | A375 |
| Glioblastoma | U-87 MG |

The foregoing paragraphs demonstrate the efficacy of the 9-amino-Doxycycline derivatives with fatty acid moieties, as anti-cancer therapeutics, and more specifically, for preventing or reducing the likelihood of metastasis. In addition to inhibiting metastasis and eradicating CSCs, compounds of the present approach also have efficacy as anti-inflammatory agents, anti-fibrotic agents, and anti-viral agents. Doxycycline was originally shown to act as an inhibitor of protein synthesis in bacteria. As a consequence, it also inhibits protein synthesis in mammalian cells as an off-target side effect.

As a result of its ability to inhibit protein synthesis, this also allows Doxycycline to act as an anti-infammatory, by reducing the synthesis and secretion of IL-6 and other cytokines, including IL-1beta and TNF-alpha, among others. Moreover, Doxycyline also inhibits fibrosis, as it can also reduce the synthesis and secretion of collagens. Finally, Doxycycline also inhibits viral replication of Dengue and other viruses, as they are made of proteins.

Compounds according to the present approach, e.g., Doxy-Myr, share many of these properties with Doxycycline, but Doxy-Myr is a more potent inhibitor of protein synthesis. Interestingly, the peri-nuclear localization pattern of Doxy-Myr in cells is reminiscent of the endoplasmic reticulum (ER), which is the major site of protein synthesis for inflammatory cytokines, collagen isoforms and viral spike glycoproteins. Therefore, the increase in potency for Doxy-Myr in reducing metastasis, may also be explained by its effect on protein synthesis in CSCs.

Importantly, the use of Doxy-Myr in fighting viral infections, by inhibiting viral replication, may have wider applicability for its use, especially in emerging viral pandemics, such as the current COVID-19 pandemic of 2020, particularly where vaccines are not yet available, or have not yet been developed.

It should be appreciated that some embodiments of the present approach may take the form of a pharmaceutical composition, such as a composition for preventing and/or reducing the likelihood of metastasis. Pharmaceutical compositions of the present approach include a 9-amino-Doxycycline derivative (including salts thereof) as an active compound, in any pharmaceutically acceptable carrier. If a solution is desired, water may be the carrier of choice for water-soluble compounds or salts. With respect to water solubility, organic vehicles, such as glycerol, propylene glycol, polyethylene glycol, or mixtures thereof, can be suitable. Additionally, methods of increasing water solubility may be used without departing from the present approach. In the latter instance, the organic vehicle can contain a substantial amount of water. The solution in either instance can then be sterilized in a suitable manner known to those in the art, and for illustration by filtration through a 0.22-micron filter. Subsequent to sterilization, the solution can be dispensed into appropriate receptacles, such as depyrogenated glass vials. The dispensing is optionally done by an aseptic method. Sterilized closures can then be placed on the vials and, if desired, the vial contents can be lyophilized. The present approach is not intended to be limited to a particular form of administration, unless otherwise stated.

In addition to the active compound, pharmaceutical formulations of the present approach can contain other additives known in the art. For example, some embodiments may include pH-adjusting agents, such as acids (e.g., hydrochloric acid), and bases or buffers (e.g., sodium acetate, sodium borate, sodium citrate, sodium gluconate, sodium lactate, and sodium phosphate). Some embodiments may include antimicrobial preservatives, such as methylparaben, propylparaben, and benzyl alcohol. An antimicrobial preservative is often included when the formulation is placed in a vial designed for multi-dose use. The pharmaceutical formulations described herein can be lyophilized using techniques well known in the art.

In embodiments involving oral administration of an active compound, the pharmaceutical composition can take the form of capsules, tablets, pills, powders, solutions, suspensions, and the like. Tablets containing various excipients such as sodium citrate, calcium carbonate and calcium phosphate may be employed along with various disintegrants such as starch (e.g., potato or tapioca starch) and certain complex silicates, together with binding agents such as polyvinylpyrrolidone, sucrose, gelatin and acacia. Additionally, lubricating agents such as magnesium stearate, sodium lauryl sulfate, and talc may be included for tableting purposes. Solid compositions of a similar type may be employed as fillers in soft and hard-filled gelatin capsules. Materials in this connection also include lactose or milk sugar, as well as high molecular weight polyethylene glycols. When aqueous suspensions and/or elixirs are desired for oral administration, the compounds of the presently disclosed subject matter can be combined with various sweetening agents, flavoring agents, coloring agents, emulsifying agents and/or suspending agents, as well as such diluents as water, ethanol, propylene glycol, glycerin and various like combinations thereof.

Additional embodiments provided herein include liposomal formulations of the active compounds disclosed herein. The technology for forming liposomal suspensions is well known in the art. When the compound is an aqueous-soluble salt, using conventional liposome technology, the same can be incorporated into lipid vesicles. In such an instance, due to the water solubility of the active compound, the active compound can be substantially entrained within the hydrophilic center or core of the liposomes. The lipid layer employed can be of any conventional composition and can either contain cholesterol or can be cholesterol-free. When the active compound of interest is water-insoluble, again employing conventional liposome formation technology, the salt can be substantially entrained within the hydrophobic lipid bilayer that forms the structure of the liposome. In either instance, the liposomes that are produced can be reduced in size, as through the use of standard sonication and homogenization techniques. The liposomal formulations comprising the active compounds disclosed herein can be lyophilized to produce a lyophilizate, which can be reconstituted with a pharmaceutically acceptable carrier, such as water, to regenerate a liposomal suspension.

With respect to pharmaceutical compositions, the pharmaceutically effective amount of an active compound described herein will be determined by the health care practitioner, and will depend on the condition, size and age of the patient, as well as the route of delivery. In one non-limited embodiment, a dosage from about 0.1 to about 200 mg/kg has therapeutic efficacy, wherein the weight ratio is the weight of the active compound, including the cases where a salt is employed, to the weight of the subject. In some embodiments, the dosage can be the amount of active compound needed to provide a serum concentration of the active compound of up to between about 1 and 5, 10, 20, 30, or 40 µM. In some embodiments, a dosage from about 1 mg/kg to about 10, and in some embodiments about 10 mg/kg to about 50 mg/kg, can be employed for oral administration. Typically, a dosage from about 0.5 mg/kg to 5 mg/kg can be employed for intramuscular injection. In some embodiments, dosages can be from about 1 µmol/kg to about 50 µmol/kg, or, optionally, between about 22 µmol/kg and about 33 µmol/kg of the compound for intravenous or oral administration. An oral dosage form can include any appropriate amount of active compound, including for example from 5 mg to, 50, 100, 200, or 500 mg per tablet or other solid dosage form.

Pharmaceutical compositions may employ an active compound as a free base or as a salt. Common salts include monohydrate and hyclate, the latter of which may be useful for improving solubility. Demonstrative pharmaceutical compositions are provided, which are meant to be non-limiting examples only. In capsule form, the composition may include 50mg or 100mg of the active compound as a base. The other ingredients may include gelatin, magnesium stearate, shellac glaze, sodium lauryl sulfate, starch, quinoline yellow (E104), erythrosine (E127), patent blue V (E131), titanium dioxide (E171), iron oxide black (E172), and propylene glycol. A delayed-release tablet form may include 60 mg or 120 mg of the active compound, and 3.6 mg or 7.2 mg, respectively, of sodium, and inactive ingredients including lactose monohydrate; microcrystalline cellulose; sodium lauryl sulfate; sodium chloride; talc; anhydrous lactose; corn starch; crospovidone; magnesium stearate; and a cellulosic polymer coating. It should be appreciated that other pharmaceutical compositions may be used without departing from the present approach, which is not intended to be limited to any specific formulation.

In some embodiments, the present approach may take the form of treatment methods comprising administering to a patient in need thereof of a pharmaceutically effective amount of a one or more pharmaceutical compositions and a pharmaceutically acceptable carrier. For example, the present approach may be used to eradicate a population of CSCs likely to cause metastasis, thereby preventing or reducing the likelihood of metastasis and recurrence from the original CSC population.

The following paragraphs describe the materials and methods used in connection with the data described herein. MCF7 and MDA-MB-231 cells were obtained from the American Type Culture Collection (ATCC). hTERT-BJ1 fibroblasts were as described in Ozsvari B, Fiorillo M, Bonuccelli G, Cappello AR, Frattaruolo L, Sotgia F, Trowbridge R, Foster R, Lisanti MP. Mitoriboscins: Mitochondrial-based therapeutics targeting cancer stem cells (CSCs), bacteria and pathogenic yeast. Oncotarget. 2017 Jul 7;8(40):67457-67472. Cells were cultured in DMEM, supplemented with 10% fetal calf serum (FCS), Glutamine and Pen/Strep.

The 9-amino-Doxycycline derivatives (e.g., Doxy-Myr, Doxy-Pal, Doxy-Laur, etc.) were custom-synthesized. Conventional peptide synthesis methods were used to covalently attach each free fatty acid to 9-amino-Doxycycline. The desired reaction products were identified chromatographically, purified, and the chemical structures were validated, by using a combination of NMR and mass spectrometry. The IUPAC names for the chemical compounds are as follows:
- Doxycycline: (4S,5S,6R,12aS)-4-(dimethylamino)-3,5,10,12,12a-pentahydroxy-6-methyl-1,11-dioxo-4a,5,5a,6-tetrahydro-4H-tetracene-2-carboxamide
- 9-Amino-Doxycycline: (45,5S,6R,12aS)-9-amino-4-(dimethylamino)-3,5,10,12,12a-pentahydroxy-6-methyl-1,11-dioxo-4a,5,5a,6-tetrahydro-4H-tetracene-2-carboxamide. Note that while it is commercially available (e.g., Frontier Scientific, Logan, UT, as item A14590, HCl), 9-Amino-Doxycycline was synthesized essentially as previously described in Barden T. C, Buckwalter B. L, Testa R. T, Petersen P. J, Lee V.J. "Glycylcyclines". 3. 9-Aminodoxycyclinecarboxamides. J. Med. Chem. 1994, 37, 3205-3211.

- Doxy-Myr: (4S,5S,6R,12aS)-4-(dimethylamino)-3,5,10,12,12a-pentahydroxy-6-methyl-1,11-dioxo-9-(tetradecanoylamino)-4a,5,5a,6-tetrahydro-4H-tetracene-2-carboxamide
- Doxy-Laur: (45,5S,6R,12aS)-4-(dimethylamino)-9-(dodecanoylamino)-3,5,10,12,12a-pentahydroxy-6-methyl-1,11-dioxo-4a,5,5a,6-tetrahydro-4H-tetracene-2-carboxamide
- Doxycycline-Pal: (4S,5S,6R,12aS)-4-(dimethylamino)-9-(hexadecanoylamino)-3,5,10,12,12a-pentahydroxy-6-methyl-1,11-dioxo-4a,5,5a,6-tetrahydro-4H-tetracene-2-carboxamide

The compounds used to generate the data discussed above were synthesized from Doxycycline hydrate, purchased from AlfaAesar. The 9-amino-Doxycycline derivatives were synthesized following the general method for (4S,5S,6R,12aS)-4-(dimethylamino)-3,5,10,12,12a-pentahydroxy-6-methyl-1,11-dioxo-9-(tetradecanoylamino)-4a,5,5a,6-tetrahydro-4H-tetracene-2-carboxamide. The following illustrates the reaction, and the individual steps are discussed below for Doxy-Myr using myristic acid (R = CH₃(CH2)₁₂). It should be appreciated that Doxy-Laur was synthesized using lauric acid (R = CH₃(CH2)₁₀), and that Doxy-Pal was synthesized using palmitic acid (R = CH₃(CH2)₁₄). Further, Doxy-TPP was synthesized using R = PH₃P⁺(CH₂)₅.

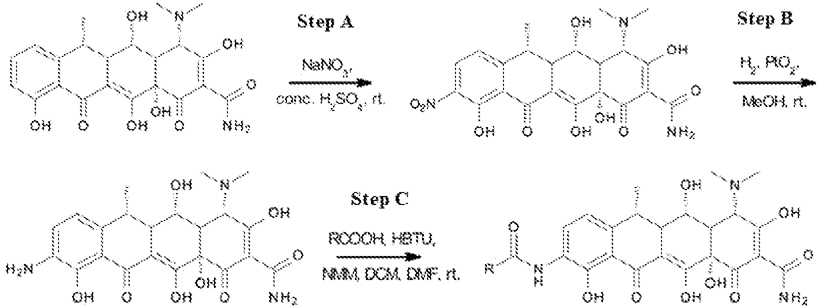

Step (a): To a stirred solution of doxycycline hydrate (1.0g, 2.16mmol) in conc. H2SO4 (5.5m1) at room temperature under nitrogen atmosphere NaNO3 (0.29g, 3.41mmol) was added and the mixture was stirred for 3 hours. The resulting dark brown oil was poured into ice cold diethyl ether (140ml), the precipitate was collected under nitrogen atmosphere, washed with diethyl ether and dried under vacuum to yield a crude 9-nitrodoxycycline.

Step (b): The crude 9-nitrodoxycycline (1.0g, 2.04mmol) was dissolved in methanol (30ml) at room temperature under nitrogen atmosphere, PtO2 (0.12g) was added and the suspension was stirred under hydrogen atmosphere for 2 hours. The catalyst was removed by filtration through Celite pad, the filtrate was poured into diethyl ether (240ml) under nitrogen atmosphere and the precipitate was collected and dried under vacuum to yield a crude 9-aminodoxycycline (0.89g, 1.94 mmol).

Step (c): The crude 9-aminodoxycycline (0.70g, 1.5mmol), myristic acid (0.36g, 1.5mmol), HBTU (0.85g, 2.25mmol) and NMM (0.33ml, 3.0mmol) in a mixture of DCM (12m1) and DMF (4ml) was stirred under nitrogen atmosphere at room temperature for 72 hours. The solvents were evaporated under reduced pressure. The resulting residue was triturated with acetonitrile (40ml), the precipitation was collected by filtration, was washed with acetonitrile (10ml), diethyl ether (20ml) and dried under vacuum. The crude product was dissolved in DMSO and purified by preparative HPLC to yield (4S,5S,6R,12aS)-4-(dimethylamino)-3,5,10,12,12a-pentahydroxy-6-methyl-1,11-dioxo-9-(tetradecanoylamino)-4a,5,5a,6-tetrahydro-4H-tetracene-2-carboxamide (1), (0.086g). 1H-NMR (MeOD) 0.89 (dd, 3H), 1.14-1.48 (m, 20H), 1.54 (d, 3H), 1.61-1.79 (m, 2H), 2.38-2.53 (dd, 2H), 2.49-2.61 (m, 2H), 2.65 (m, 8H), 3.68 (dd, 1H), 3.94 (m, 1H), 6.93 (d, 1H), 8.14 (d, 1H). LC-MS 670.2 [M+H]+, RT 2.78min.

For Doxy-Laur, (4S,5S,6R,12aS)-4-(dimethylamino)-9-(dodecanoylamino)-3,5,10,12,12a-pentahydroxy-6-methyl-1,11-dioxo-4a,5,5a,6-tetrahydro-4H-tetracene-2-carboxamide (2). LC-MS 642.1 [M+H]+, RT 2.42min.

For Doxy-Pal, (4S,5S,6R,12aS)-4-(dimethylamino)-9-(hexadecanoylamino)-3,5,10,12,12a-pentahydroxy-6-methyl-1,11-dioxo-4a,5,5a,6-tetrahydro-4H-tetracene-2-carboxamide (3).LC-MS 698.2 [M+H]+, RT 3.02min.

For Doxy-TPP, [6-[[(5R,6S,7S,10aS)-9-carbamoyl-7-(dimethylamino)-1,6,8,10a,11-pentahydroxy-5-methyl-10,12-dioxo-5a,6,6a,7-tetrahydro-5H-tetracen-2-yl]amino]-6-oxo-hexyl]-triphenyl-phosphonium oxalate (4). LC-MS 409.7 [M½]+, RT 1.53min.

For the 3D-Mammosphere Assay, a single cell suspension of MCF7 cells was prepared using enzymatic (1x Trypsin-EDTA, Sigma Aldrich) and manual disaggregation (25-gauge needle). Cells were then plated at a density of 500 cells/cm² in mammosphere medium (DMEM-F12/B27/ EGF (20-ng/ml) /PenStrep) in non-adherent conditions, in culture dishes coated with (2-hydroxyethylmethacrylate) (poly-HEMA, Sigma). Cells were grown for 5 days and maintained in a humidified incubator at 37°C at an atmospheric pressure in 5% (v/v) carbon dioxide/air. After 5 days in culture, spheres >50 µm were counted using an eye-piece graticule, and the percentage of cells plated which formed spheres was calculated and is referred to as percent mammosphere formation, normalized to vehicle-alone treated controls. Mammosphere assays were performed in triplicate and repeated three times independently.

Fluorescence Imaging: Fluorescent images were taken after 72 hours of incubation of MCF7 cells treated with either Doxycycline or Doxy-Myr (both at 10 µM), or vehicle control. Cell cultures were imaged with the EVOS Cell Imaging System (Thermo Fisher Scientific, Inc.), using the GFP channel. No fluorescent dye was used before imaging, therefore, any changes in signal were exclusively due to the auto-fluorescent nature of the Doxycycline compounds.

Cell Viability Assay: The Sulphorhodamine (SRB) assay is based on the measurement of cellular protein content. After treatment for 72h in 96-well plates, (8,000 cells/well), cells were fixed with 10% trichloroacetic acid (TCA) for 1 hour in the cold room, and were dried overnight at room temperature. Then, cells were incubated with SRB for 15 min, washed twice with 1% acetic acid, and air dried for at least 1 hour. Finally, the protein-bound dye was dissolved in a 10 mM Tris, pH 8.8, solution and read using the plate reader at 540-nm.

Cell Proliferation: Briefly, MCF7 cells or hTERT-BJ1 fibroblasts were seeded in each well (10,000 cells/well) and employed to assess the efficacy of Doxycycline and Doxy-Myr, using RTCA (real-time cell analysis), via the measurement of cell-induced electrical impedance plate (Acea Biosciences Inc.). This approach allows the quantification of the onset and kinetics of the cellular response. Experiments were repeated several times independently, using quadruplicate samples for each condition.

Cell Cycle Analysis: Performed on MCF7 cells treated with Doxycycline, Doxy-Myr or vehicle-alone. Briefly, after trypsinization, the re-suspended cells were incubated with 10 ng/ml of Hoechst solution (Thermo Fisher Scientific) for 40 min at 37°C under dark conditions. Following a 40 min period, the cells were washed and re-suspended in PBS Ca/Mg for acquisition on the Attune NxT flow cytometer (Thermo Scientific). 10,000 events per condition were analyzed. Gated cells were manually-categorized into cell-cycle stages.

Bacterial Growth Assays: Briefly, antibiotic activity was assessed using standard assay systems. The antibiotic activity of Doxycycline analogues was determined experimentally, using Resazurin (R7017; Sigma-Aldrich, Inc.) as a probe, in a 96-well plate format, using standard strains of E. *coli* and S. *aureus.* The minimum inhibitory concentration (MIC) for the studied compounds was determined using the broth microdilution method, the reference susceptibility test for rapidly growing aerobic or facultative microorganism. The assays were performed according to the Clinical and Laboratory Standards Institute (CLSI) guidelines. The test compounds and positive control (doxycycline, Sigma Aldrich #D1822) stock solutions were prepared at 25 mM in DMSO and serially diluted (2-fold dilution from 200 - 1.56 µM) in cation adjusted Mueller Hinton Broth (MHB, Sigma Aldrich #90922) in 96 well transparent plates (VWR #734-2781) into a final volume of 50 µL/well. *Staphylococcus aureus* (ATCC 29213) and E. *coli* (ATCC 25922) cultures were grown overnight at 37 °C in Mueller Hinton Agar (MHA, Sigma Aldrich #70191). A single colony of each strain was then grown overnight at 37 °C in MHB until OD₆₀₀ ~ 0.6-0.8 and further diluted into MHB to a concentration of 106 colony forming units (CFU)/mL, which was equivalent to an OD₆₀₀ ~ 0.01. Then, 50 µL of the diluted inoculums was transferred to the wells of the previously prepared 96-well plates containing the test compounds, negative control (1% DMSO in MHB) and positive control (doxycycline). Final wells volume was 100 µL, final concentrations for the testing compounds were between 100 - 0.78 µM and final microorganism concentration was 5 x 10⁵ CFU/mL. Subsequently, 10 uL of one negative control well was plated in a petri dish containing MHA to check CFU and the purity of the cultures. The plates were incubated at 37 °C for 24 h after which 20 µL of resazurin solution (0.2 mg/mL) was added to the wells followed by 1h30min incubation at 37 °C. The OD₅₇₀ and OD₆₀₀ were measured in a microplate reader (BMG FLUOstar Omega). The ratio between OD₅₇₀ and OD₆₀₀ was determined and the MIC represents the lowest concentration of compound that inhibited bacterial growth (OD₅₇₀/OD₆₀₀ ratio inferior to the average ratio determined for negative control wells). MIC values were determined by three independent experiments.

Assays for Tumor Growth, Metastasis and Embryo Toxicity: These xenograft assays were carried out without any major modifications.
a) Preparation of Chicken Embryos. Fertilized White Leghorn eggs were incubated at 37.5°C with 50% relative humidity for 9 days. At that moment (E9), the chorioallantoic membrane (CAM) was dropped down by drilling a small hole through the eggshell into the air sac, and a 1 cm² window was cut in the eggshell above the CAM.
b) Amplification and Grafting of Tumor Cells. The MDA-MB-231 tumor cell line was cultivated in DMEM medium supplemented with 10% FBS and 1% penicillin/streptomycin. On day E9, cells were detached with trypsin, washed with complete medium and suspended in graft medium. An inoculum of 1 X 10⁶ cells was added onto the CAM of each egg (E9) and then eggs were randomized into groups.
c) Tumor Growth Assays. At day 18 (E18), the upper portion of the CAM was removed from each egg, washed in PBS and then directly transferred to paraformaldehyde (fixation for 48 h) and weighed. For tumor growth assays, at least 10 tumor samples were collected and analysed per group (n ≥ 10).
d) Metastasis Assays. On day E18, a 1 cm² portion of the lower CAM was collected to evaluate the number of metastatic cells in 8 samples per group (n=8). Genomic DNA was extracted from the CAM (commercial kit) and analyzed by qPCR with specific primers for Human Alu sequences. Calculation of Cq for each sample, mean Cq and relative amounts of metastases for each group are directly managed by the Bio-Rad^{®} CFX Maestro software. A one-way ANOVA analysis with post-tests was performed on all the data.
e) Embryo Tolerability Assay. Before each administration, the treatment tolerability was evaluated by scoring the number of dead embryos.

Statistical Analysis: Statistical significance was determined using the Student's t-test, values of less than 0.05 were considered significant. Data are shown as the mean ± SEM, unless stated otherwise.

The terminology used in the description of embodiments of the present approach is for the purpose of describing particular embodiments only and is not intended to be limiting. As used in the description and the appended claims, the singular forms "a," "an" and "the" are intended to include the plural forms as well, unless the context clearly indicates otherwise. The present approach encompasses numerous alternatives, modifications, and equivalents as will become apparent from consideration of the following detailed description.

It will be understood that although the terms "first," "second," "third," "a)," "b)," and "c)," etc. may be used herein to describe various elements of the present approach, and the claims should not be limited by these terms. These terms are only used to distinguish one element of the present approach from another. Thus, a first element discussed below could be termed an element aspect, and similarly, a third without departing from the teachings of the present approach. Thus, the terms "first," "second," "third," "a)," "b)," and "c)," etc. are not intended to necessarily convey a sequence or other hierarchy to the associated elements but are used for identification purposes only. The sequence of operations (or steps) is not limited to the order presented in the claims.

Unless otherwise defined, all terms (including technical and scientific terms) used herein have the same meaning as commonly understood by one of ordinary skill in the art. It will be further understood that terms, such as those defined in commonly used dictionaries, should be interpreted as having a meaning that is consistent with their meaning in the context of the present application and relevant art and should not be interpreted in an idealized or overly formal sense unless expressly so defined herein. All publications, patent applications, patents and other references mentioned herein are incorporated by reference in their entirety. In case of a conflict in terminology, the present specification is controlling.

Also, as used herein, "and/or" refers to and encompasses any and all possible combinations of one or more of the associated listed items, as well as the lack of combinations when interpreted in the alternative ("or").

Unless the context indicates otherwise, it is specifically intended that the various features of the present approach described herein can be used in any combination. Moreover, the present approach also contemplates that in some embodiments, any feature or combination of features described with respect to demonstrative embodiments can be excluded or omitted.

As used herein, the transitional phrase "consisting essentially of" (and grammatical variants) is to be interpreted as encompassing the recited materials or steps "and those that do not materially affect the basic and novel characteristic(s)" of the claim. Thus, the term "consisting essentially of" as used herein should not be interpreted as equivalent to "comprising."

The term "about," as used herein when referring to a measurable value, such as, for example, an amount or concentration and the like, is meant to encompass variations of ± 20%, ± 10%, ± 5%, ± 1%, ± 0.5%, or even ± 0.1% of the specified amount. A range provided herein for a measurable value may include any other range and/or individual value therein.

Having thus described certain embodiments of the present approach, it is to be understood that the scope of the appended claims is not to be limited by particular details set forth in the above description as many apparent variations thereof are possible without departing from the spirit or scope thereof as hereinafter claimed.
The present application also includes the following numbered clauses:
1. A compound having the general formula: 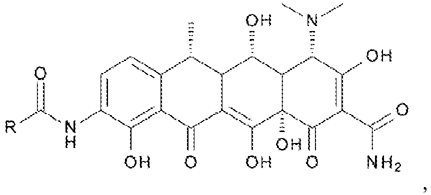 wherein R comprises a linear, saturated alkyl having from 4 to 18 carbons, or a pharmaceutically acceptable salt thereof.
2. The compound of clause 1, wherein R comprises a linear, saturated alkyl having from 11 to 16 carbons.
3. The compound of clause 1, wherein the compound is 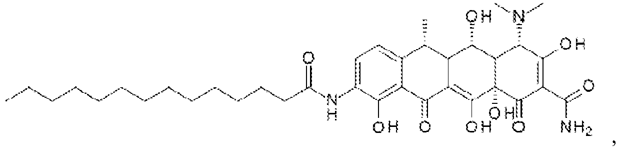 or a pharmaceutically acceptable salt thereof.
4. The compound of clause 1, wherein the compound is 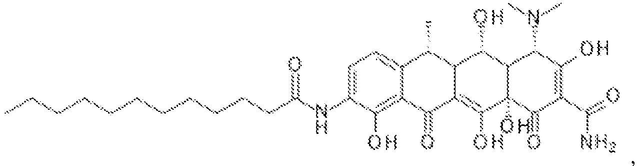 or a pharmaceutically acceptable salt thereof.
5. The compound of clause 1, wherein the compound is 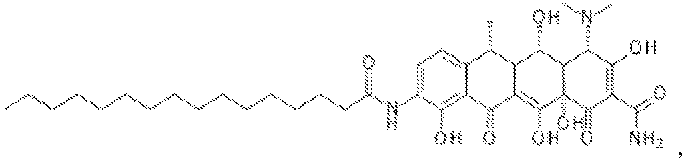 or a pharmaceutically acceptable salt thereof.
6. The compound of clause 1, comprising a pharmaceutically acceptable salt, and wherein the salt is one of monohydrate and hyclate.
7. A pharmaceutical composition for preventing metastasis, the composition comprising a compound having the general formula: 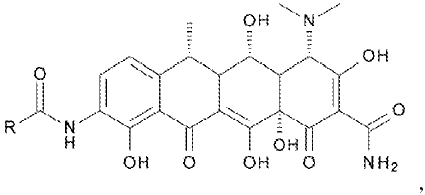 wherein R comprises a linear, saturated alkyl having from 4 to 18 carbons, or a pharmaceutically acceptable salt thereof, and a pharmaceutically acceptable carrier.
8. The pharmaceutical composition of clause 7, wherein R comprises a linear, saturated alkyl having from 11 to 16 carbons.
9. The pharmaceutical composition of clause 7, wherein the compound is 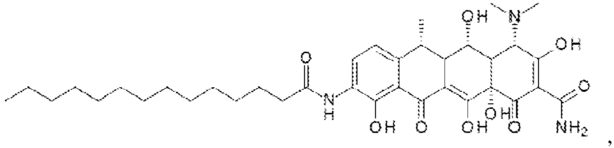 or a pharmaceutically acceptable salt thereof.
10. The pharmaceutical composition of clause 7, wherein the compound is 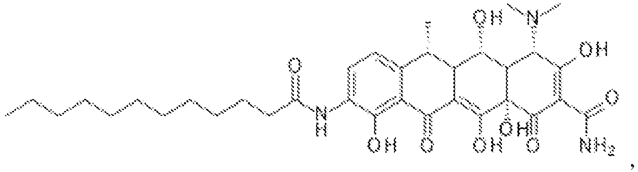 or a pharmaceutically acceptable salt thereof.
11. The pharmaceutical composition of clause 7, wherein the compound 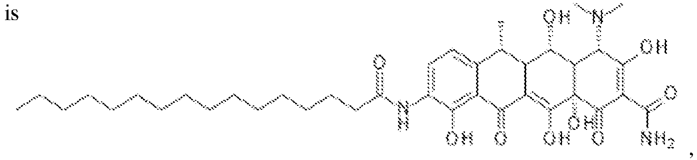 or a pharmaceutically acceptable salt thereof.
12. The pharmaceutical composition of clause 7, comprising a pharmaceutically acceptable salt, and wherein the salt is one of monohydrate and hyclate.
13. The pharmaceutical composition of clause 7, wherein the pharmaceutically acceptable carrier comprises at least one of a sugar, a starch, cellulose, an excipient, an oil, a glycol, a polyol, an ester, an agar, and a buffering agent.
14. The pharmaceutical composition of any of clauses 7-13, for use in one of preventing metastasis, reducing inflammation, reducing fibrosis, and reducing viral replication.
15. A method for preventing metastasis in a patient, the method comprising administering to the patient a pharmaceutically effective amount of a pharmaceutical composition of any of clauses 7-13.
16. A method for reducing inflammation in a patient, the method comprising administering to the patient a pharmaceutically effective amount of a pharmaceutical composition of any of clauses 7-13.
17. A method for reducing fibrosis in a patient, the method comprising administering to the patient a pharmaceutically effective amount of a pharmaceutical composition of any of clauses 7-13.
18. A method for reducing virus replication in a patient, the method comprising administering to the patient a pharmaceutically effective amount of a pharmaceutical composition of any of clauses 7-13.

## Claims

1. A compound for use in a method of treating breast cancer in a patient, the compound having the general formula: 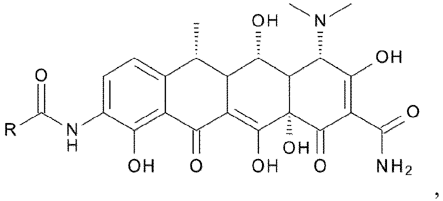 wherein R comprises a linear, saturated alkyl having from 4 to 18 carbons, the compound comprising a pharmaceutically acceptable salt, wherein the salt is one of monohydrate and hyclate.

2. A pharmaceutical composition for use in a method of treating breast cancer in a patient, wherein the pharmaceutical composition comprises:
(a) a compound having the general formula: 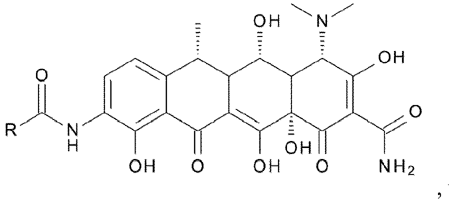 wherein R comprises a linear, saturated alkyl having from 4 to 18 carbons, the compound comprising a pharmaceutically acceptable salt, wherein the salt is one of monohydrate and hyclate; and
(b) a pharmaceutically acceptable carrier.

3. The compound for use according to claim 1 or the pharmaceutical composition for use according to claim 2, wherein R comprises a linear, saturated alkyl having from 11 to 16 carbons.

4. The compound for use according to claim 1 or the pharmaceutical composition for use according to claim 2, wherein the compound is

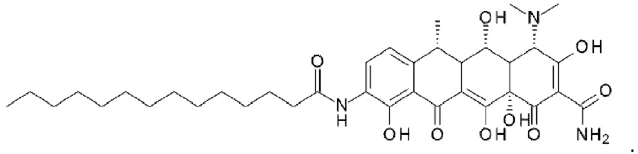

5. The compound for use according to claim 1 or the pharmaceutical composition for use according to claim 2, wherein the compound is

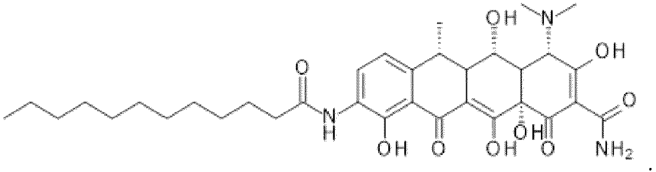

6. The compound for use according to claim 1 or the pharmaceutical composition for use according to claim 2, wherein the compound is

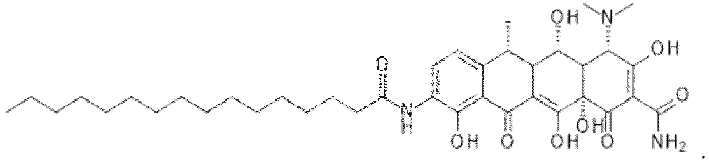

7. The pharmaceutical composition for use of any one of claims 2-6, wherein the pharmaceutically acceptable carrier comprises at least one of: a sugar, a starch, cellulose, an excipient, an oil, a glycol, a polyol, an ester, an agar, and a buffering agent.

8. The compound for use according to any one of claims 1-6 or the pharmaceutical composition for use according to any one of claims 2-7, for use in a method of preventing or inhibiting tumor cell metastasis in breast cancer.

9. The compound for use according to any one of claims 1-6 or the pharmaceutical composition for use according to any one of claims 2-7, for use in inhibiting the anchorage-independent propagation of breast cancer stem cells.

10. The compound for use according to any one of claims 1-6, 8 or 9 or the pharmaceutical composition for use according to any one of claims 2-9, wherein the breast cancer is ER+ breast cancer.

11. The compound for use according to any one of claims 1-6, 8 or 9 or the pharmaceutical composition for use according to any one of claims 2-9, wherein the breast cancer is ER- breast cancer.
